# EUROPEAN PATENT SPECIFICATION

(11) **EP 3 551 187 B1**
(45) Date of publication and mention of the grant of the patent: **17.02.2021**
(21) Application number: 17811550.7
(22) Date of filing: 06.12.2017
(51) Int. Cl.: A61K 31/496, A61K 31/538, A61P 11/00

(54) **NINTEDANIB FOR USE IN METHODS FOR THE TREATMENT OF INTERSTITIAL LUNG DISEASES BY COADMINISTRATION WITH OLODATEROL**
NINTEDANIB ZUR VERWENDUNG BEI VERFAHREN ZUR BEHANDLUNG VON INTERSTITIELLEN LUNGENERKRANKUNGEN DURCH GEMEINSAME VERABREICHUNG MIT OLODATEROL
NINTÉDANIB DESTINÉ À ÊTRE UTILISÉ DANS DES MÉTHODES DE TRAITEMENT DE MALADIES PULMONAIRES INTERSTITIELLES PAR CO-ADMINISTRATION D'OLODATÉROL

(30) Priority: 12.12.2016 EP 16203359
(43) Date of publication of application: 16.10.2019
(73) Proprietor: Boehringer Ingelheim International GmbH, 55216 Ingelheim am Rhein (DE)
(72) Inventor: WOLLIN, Stefan, 55216 Ingelheim am Rhein (DE); WEX, Eva, 55216 Ingelheim am Rhein (DE); HERRMANN, Franziska, 55216 Ingelheim am Rhein (DE)
(74) Representative: Simon, Elke Anna Maria
(86) International application number: PCT/EP2017/081691
(87) International publication number: WO 2018/108669

(56) References cited:
- Herrmann Franziska Helena: "Olodaterol in model systems of fibrosis (Dissertation)", , 20 June 2017 (2017-06-20), pages FP-VIII,1-115, XP002777561, Universität Konstanz Retrieved from the Internet: URL:https://kops.uni-konstanz.de/bitstream /handle/123456789/39671/Herrmann_0-415928. pdf?sequence=3 [retrieved on 2018-01-23]
- SIRI T. LEHTONEN ET AL: "Pirfenidone and nintedanib modulate properties of fibroblasts and myofibroblasts in idiopathic pulmonary fibrosis", RESPIRATORY RESEARCH, vol. 17, no. 1, 14, 4 February 2016 (2016-02-04), XP055394528, GB ISSN: 1465-993X, DOI: 10.1186/s12931-016-0328-5
- LUTZ WOLLIN ET AL: "Mode of action of nintedanib in the treatment of idiopathic pulmonary fibrosis", EUROPEAN RESPIRATORY JOURNAL., vol. 45, no. 5, 30 May 2015 (2015-05-30), pages 1434-1445, XP055341429, DK ISSN: 0903-1936, DOI: 10.1183/09031936.00174914
- Herrmann Franziska: "Olodaterol attenuates bleomycin induced lung fibrosis", European Respiratory Journal, vol. 48, no. Suppl. 60 8 November 2016 (2016-11-08), XP002777562, ISSN: 1399-3003, DOI: 10.1183/13993003.congress-2016.PA5056 Retrieved from the Internet: URL:http://erj.ersjournals.com/content/48/ suppl_60/PA5056 [retrieved on 2018-01-23]
- HERRMANN FRANZISKA ELENA ET AL: "Olodaterol shows anti-fibrotic efficacy in in vitro and in vivo models of pulmonary fibrosis.", BRITISH JOURNAL OF PHARMACOLOGY, vol. 174, no. 21, November 2017 (2017-11), pages 3848-3864, XP002777563, ISSN: 1476-5381, DOI: 10.1111/bph.13982

## Description

### Field of the Invention

This invention relates to nintedanib for use in methods for the treatment of interstitial lung diseases by coadministration with olodaterol. In addition, the invention relates to pharmaceutical compositions comprising said compounds and to pharmaceutical kits comprising said compositions.

### Background of the Invention

Interstitial lung diseases (ILD) include a large and diverse group of more than 200 lung diseases and respiratory conditions characterized by inflammation and fibrosis of the interstitium, the tissue between the air sacs of the lung (see, for instance, du Bois, Nat. Rev. Drug Discov. 2010, 9, 129-140).

One of the most common types of ILD is idiopathic pulmonary fibrosis (IPF). Other ILDs with similar pathological fibrotic alterations in the lung interstitium and a progressive fibrosis include connective tissue disease-associated ILD (CTD-ILD) which is mainly systemic sclerosis-ILD (SSc-ILD) and rheumatoid arthritis-ILD (RA-ILD), idiopathic non-specific interstitial pneumonia (iNSIP), chronic hypersensitivity pneumonitis (CHP), unclassifiable idiopathic interstitial pneumonia (IIP), interstitial pneumonia with autoimmune features (IPAF) and environmental/occupational fibrosing lung diseases like asbestosis and silicosis.

IPF is a rare disease of unknown aetiology and poor prognosis that is characterized by progressive fibrosis of the interstitium of the lung, leading to decreasing lung volume and progressive pulmonary insufficiency. The lung function in patients with lung fibrosis is determined as forced vital capacity (FVC).

Recently, drugs containing pirfenidone or nintedanib as active pharmaceutical ingredients were approved for the treatment of IPF.

Fibroblasts play a central role in the pathogenesis of fibrotic processes, and several factors influence their proliferation and extracellular matrix (ECM) synthesis. In ILDs, these mesenchymal cells have an increased activity with respect to proliferation, migration ECM synthesis and response to fibrogenic cytokines. The increased deposition of ECM from activated fibroblasts (myofibroblasts) contributes to the stiffening of the lung tissue and the destruction of alveolar oxygen exchange area which results in progressive dyspnea and eventually death.

Endothelin-1 (ET-1) is an important mediator in the pathogenesis of pulmonary fibrosis. ET-1 is a 21-amino acid peptide which has potent vasoconstrictive, bronchoconstrictive, and mitogenic activities (Gallelli et al., J. Cell Biochem. 2005, 96, 858-868.). In the lung, fibroblasts, endothelial cells, alveolar macrophages, and epithelial cells are the major sources of ET-1. In addition to its well-known contribution to pulmonary hypertension (Ahmedat et al., Eur. J. Pharmacol. 2012, 691, 218-224), it exerts various pro-fibrotic functions, like enhancing fibroblast resistance to apoptosis, cytokine release, fibroblast proliferation and inducing the expression of matrix associated genes and ECM proteins. Moreover, ET-1 cooperates with TGF-β to exert its pro-fibrotic activity (Swigris et al., BioDrugs. 2010, 24, 49-54.).

Nintedanib, the compound of formula A, 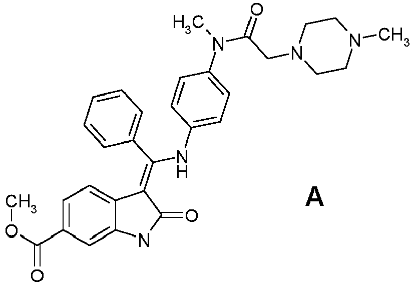 is described in WO 01/27081. WO 2004/013099 discloses its monoethanesulphonate salt; further salt forms are presented in WO 2007/141283.

Pharmaceutical dosage forms comprising nintedanib are disclosed in WO 2009/147212 and in WO 2009/147220. Also, a dry powder formulation for inhalation has been described (Vartiainen et al., poster presentation at the International Colloquium of Lung and Airway Fibrosis in Dublin, September 2016).

The use of nintedanib for the treatment of fibrotic diseases is described in WO 2006/067165.

Nintedanib is a highly potent, orally bioavailable inhibitor of vascular endothelial growth factor receptors (VEGFRs), platelet-derived growth factor receptors (PDGFRs) and fibroblast growth factor receptors (FGFRs). It binds competitively to the adenosine triphosphate (ATP) binding pocket of these receptors and blocks intracellular signalling. In addition, nintedanib inhibits Fms-like tyrosine-protein kinase 3 (Flt 3), lymphocyte-specific tyrosine-protein kinase (Lck), tyrosine-protein kinase lyn (Lyn) and protooncogene tyrosine-protein kinase src (Src) (Hilberg et al., Cancer Res. 2008, 68, 4774-4782).

Nintedanib was shown to be able to inhibit or attenuate cellular proliferation, contributing to angiogenesis (Hilberg et al., Cancer Res. 2008, 68, 4774-4782), as well as lung fibroblast proliferation, migration (Hostettler et al., Respir Res. 2014, 15, 157) and transformation to myofibroblasts (Wollin et al., Eur. Respir J 2015, 45, 1434-1445.) contributing to lung fibrosis (e.g. IPF), SSc-ILD and RA-ILD. Furthermore, it revealed anti-fibrotic and anti-inflammatory activity in lung fibrosis models (Wollin et al., Eur. Respir J 2015, 45, 1434-1445; Wollin et al., J. Pharmacol. Exp. Ther. 2014, 394, 209-220). Nintedanib demonstrated the ability to inhibit fibroblast migration, proliferation and transformation to myofibroblasts in SSc cellular models, to attenuate skin and lung fibrosis in SSc animal models (Huang et al., Ann. Rheum. Dis. 2016, 74, 883-890) and to reduce lung fibrosis in RA-ILD animal models (Redente et al., Am J Respir Crit Care Med 2016, 193, A4170).

Positive results, in terms of rate reduction of decline in FVC, were obtained for the use of nintedanib in the treatment of patients with IPF in clinical trials (Richeldi et al., N. Engl. J. Med. 2014, 370, 2071-2082; Richeldi et al., N. Engl. J. Med. 2011, 365, 1079-1087).

Olodaterol, the compound of formula B, 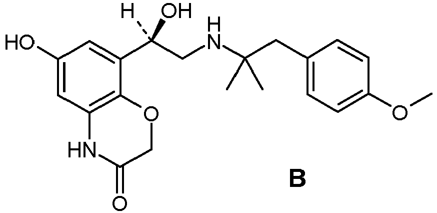 salts thereof as well as the use in respiratory diseases are disclosed in WO 2004/045618, WO 2005/111005 and WO 2005/102350.

The preparation of olodaterol is presented in WO 2007/020227 and WO 2008/090193. WO 2005/110421, WO 2005/110359, WO 2007/042468, WO 2010/057927 and WO 2010/057928 describe pharmaceutical formulations of olodaterol.

Olodaterol is a once-daily β2-adrenoceptor agonist approved for the treatment of chronic obstructive pulmonary disease (COPD).

It is also known that the pro-fibrotic activity of lung fibroblasts may be suppressed by β-adrenoceptor activation (Lamyel et al., Naunyn-Schmiedeberg's Arch Pharmacol 2011, 384, 133-145). Olodaterol was shown to attenuate pro-fibrotic events in primary human lung fibroblasts (Herrmann et al., European Respiratory Journal 2014, 44, P878) and to have anti-fibrotic properties in a mouse model of lung fibrosis (Herrmann et al., poster presentation at the European Respiratory Society International Congress in London, September 2016).

### Brief Description of the Drawings

FIG 1:
   Inhibition of TGF-β induced ET-1 release in cultured human lung fibroblasts by incubation with 1 pM olodaterol (left bar), 30 nM nintedanib (right bar) and with the combination of both (middle bar), normalized to unstimulated ET-1 levels (= 100% inhibition = dotted line) and TGF-β-stimulated ET-1 levels (= 0% inhibition)
FIG 2:
   Inhibition of TGF-β induced ET-1 release in cultured human lung fibroblasts by incubation with 10 pM olodaterol (left bar), 30 nM nintedanib (right bar) and with the combination of both (middle bar), normalized to unstimulated ET-1 levels (= 100% inhibition = dotted line) and TGF-β-stimulated ET-1 levels (= 0% inhibition)
FIG 3:
Inhibition of TGF-β induced ET-1 release in cultured human lung fibroblasts by incubation with 100 pM olodaterol (left bar), 30 nM nintedanib (right bar) and with the combination of both (middle bar), normalized to unstimulated ET-1 levels (= 100% inhibition = dotted line) and TGF-β-stimulated ET-1 levels (= 0% inhibition)

### Summary of the Invention

The present invention is defined in the appended claims. In a first aspect, the present invention relates to a tyrosine kinase inhibitor, selected from nintedanib and pharmaceutically acceptable salts thereof, for use in a method for the treatment of one or more interstitial lung diseases in a patient in need thereof by coadministration with a β2-adrenoceptor agonist, selected from olodaterol and pharmaceutically acceptable salts thereof.

In a second aspect, the present invention relates to a pharmaceutical composition for inhalative administration, selected from aerosol compositions, dry powders, suspensions and solutions, comprising said tyrosine kinase inhibitor and said β2-adrenoceptor agonist to be coadministered.

In a third aspect, the present invention relates to pharmaceutical kits, comprising one or more pharmaceutical compositions and a medical device for their inhalative administration, for simultaneous, sequential and/or separate use of said active ingredients. Other aspects of the present invention will become apparent to the person skilled in the art directly from the foregoing and following description.

### General Terms and Definitions

Terms not specifically defined herein should be given the meanings that would be given to them by one of skill in the art in light of the disclosure and the context. As used in the specification, however, unless specified to the contrary, the following terms have the meaning indicated and the following conventions are adhered to.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, and commensurate with a reasonable benefit/risk ratio.

As used herein, "pharmaceutically acceptable salt" refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like.

The terms "treatment" and "treating" as used herein embrace both therapeutic, i.e. curative and/or palliative, and preventive, i.e. prophylactic, treatment.

Therapeutic treatment refers to the treatment of patients having already developed one or more of said conditions in manifest, acute or chronic form. Therapeutic treatment may be symptomatic treatment in order to relieve the symptoms of the specific indication or causal treatment in order to reverse or partially reverse the conditions of the indication or to stop or slow down progression of the disease.

Preventive treatment ("prevention") refers to the treatment of patients at risk of developing one or more of said conditions, prior to the clinical onset of the disease in order to reduce said risk.

The terms "treatment" and "treating" include the administration of one or more active compounds in order to prevent or delay the onset of the symptoms or complications and to prevent or delay the development of the disease, condition or disorder and/or in order to eliminate or control the disease, condition or disorder as well as to alleviate the symptoms or complications associated with the disease, condition or disorder.

The term "therapeutically effective amount" means an amount of a compound of the present invention that (i) treats or prevents the particular disease or condition, (ii) attenuates, ameliorates, or eliminates one or more symptoms of the particular disease or condition, or (iii) prevents or delays the onset of one or more symptoms of the particular disease or condition described herein.

### Detailed Description of the Invention

The present invention allows for a more efficient treatment of patients with ILDs, in particular with reduced systemic side effects.

In a first aspect of the present invention, it is surprisingly found that the tyrosine kinase inhibitor nintedanib and the β2-adrenoceptor agonist olodaterol, when coadministered, exert synergistic anti-fibrotic effects.

As could be demonstrated, cultured human lung fibroblasts (HLF) from patients with IPF release the fibrotic mitogen ET-1 upon stimulation with TGF-β, an important mediator of fibrotic processes:
The principle of the assay is the incubation of IPF-HLF with transforming growth factor beta (TGF-β) for 48 h. In the cell culture supernatant the concentration of the pro-fibrotic mediator endothelin-1 (ET-1) was determined by enzyme linked immune sorbent assay.
HLFs from 5 different patients with idiopathic pulmonary fibrosis (IPF) were used to do repeated measurements. For the control groups without TGF-β stimulation, data sets from 11 incubations were included and for TGF-β-stimulated fibroblasts data sets from 12 incubations were included. For all olodaterol and/or nintedanib-treated groups, n = 6 incubations were conducted. The experiments were conducted in the presence of nintedanib at 30 nM concentration and/or olodaterol at 1 pM (FIG 1), 10 pM (FIG 2) and 100 pM (FIG 3) concentrations, respectively.

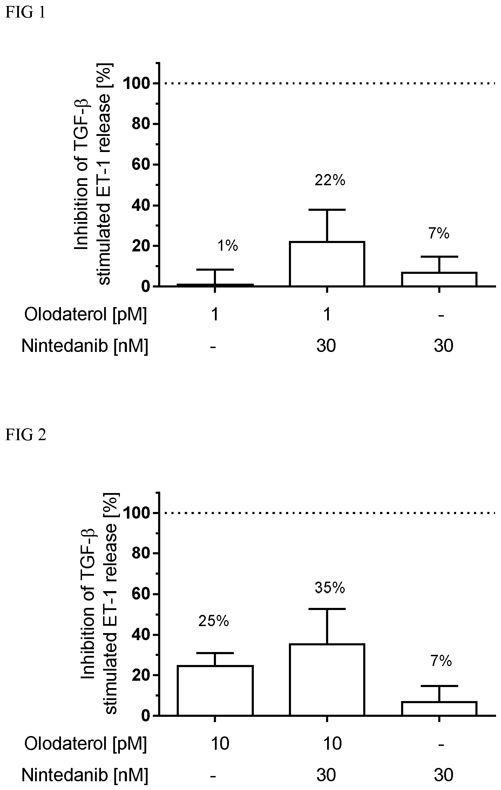

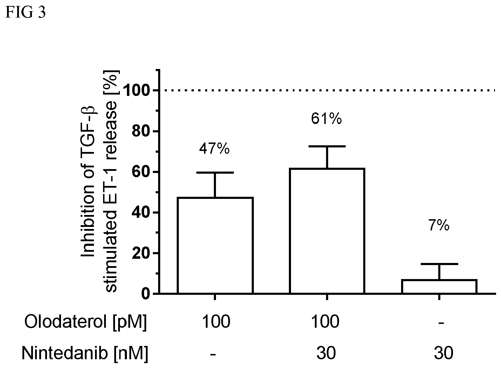

Incubation with olodaterol at concentrations of 1 - 100 pM reduces the ET-1 release by 1 % - 47 % (FIG 1 to 3). Although the incubation with nintedanib at human relevant concentrations of 30 nM does not reduce the ET-1 release substantially (7 %, FIG 1 to 3), the combined use of nintedanib and olodaterol results in a synergistic increase in the inhibitory capacity up to 22 % - 61 % (FIG 1 to 3).

The combination of nintedanib and olodaterol may thus be used beneficially for the treatment of ILDs.

Therapeutically effective doses of nintedanib, when applied orally to a patient in need thereof, are in the range from 100 mg to 300 mg per day, preferably twice daily application of 100 mg or 150 mg, approximately 12 hours apart. When applied via inhalation, therapeutically effective daily doses of nintedanib are in the range from 10 mg to 100 mg. Therapeutically effective doses of olodaterol, when applied via inhalation, will be in the range from 0.01 µg to 50 µg per day.

The actual therapeutically effective amount or therapeutic dosage will of course depend on factors known by those skilled in the art such as age and weight of the patient, route of administration and severity of disease. In any case, the combination will be administered at dosages and in a manner which allows a therapeutically effective amount to be delivered based upon a patient's unique condition. Likewise, the determination of the necessity of dose adjustments, e.g. due to adverse reactions to the active pharmaceutical ingredient, and their putting into practice will be known to the one skilled in the art.

In a second aspect, the present disclosure describes that pharmaceutical compositions of nintedanib may be formulated that are suitable for inhalative administration. Thus, pharmaceutical compositions for inhalative administration that comprise both nintedanib and olodaterol are within the scope of the present invention. Said compositions may be used advantageously for the treatment of ILDs in patients in need thereof.

In IPF patients, nintedanib administered twice daily as an oral formulation slows down disease progression. This oral application may be combined advantageously with inhalative administration of olodaterol. However, nintedanib is frequently associated with systemic adverse events like diarrhea, nausea, vomiting and weight loss, which may limit the effective dosage and may lead to patient incompliance (Richeldi et al., N. Engl. J. Med. 2014, 370, 2071-2082).

It will thus be beneficial to administer also nintedanib via inhalation. In fact, it is found that nintedanib may reveal the same inhibitory activity when administered orally or via inhalation. The combined topical administration of nintedanib and olodaterol to the lung as an inhaled formulation is therefore expected on the one hand to exert greater efficacy in preserving lung function, e.g. resulting in an improved reduction of the FVC decline, in patients with ILDs. On the other hand, the inhalative route of administration is expected to improve the adverse event profile in those patients. This is due to the fact that the daily dose of nintedanib may be lowered in a combination therapy and that the oral administration route, to which a number of negative side effects are linked, may be avoided.

Suitable preparations for administering the active pharmaceutical ingredients of the present invention will be apparent to those with ordinary skill in the art and include for example tablets, pills, capsules, suppositories, lozenges, troches, solutions, syrups, elixirs, sachets, injectables, inhalatives and powders etc.. Suitable tablets may be obtained, for example, by mixing one or more of the above-mentioned active pharmaceutical ingredients with known excipients, for example inert diluents, carriers, disintegrants, adjuvants, surfactants, binders and/or lubricants.

In particular, the compositions of the present invention may be suitable for inhalation, for example, in the form of aerosol compositions, inhalable powders, sprays, solutions, suspensions, nasal sprays, nasal drops or insufflation powders, preferably propellant-containing metered-dose aerosols, dry powders for inhalation and propellant-free inhalable suspensions or solutions, including concentrates or sterile ready-to-use inhalable solutions. The various dosage forms according to the present invention may comprise pharmaceutical carriers/excipients suitable for formulating the same in order to, for example, stabilize the drug formulation and prolong its shelf life, such as stabilizers, antioxidants, lubricants and pH adjusters. Preferably, the compositions according to the present invention are formulated as aqueous-ethanolic solutions with or without surface active (i.e. surface tension lowering) pharmaceutical excipients, for example tensides and/or surfactants, that may improve distribution and tissue penetration throughout the lung.

The above-mentioned pharmaceutical compositions for inhalation may be administered by any suitable methods used for delivery of the drugs to the respiratory tract. For example, said compositions may be administered by any conventional means, such as using a metered dose inhaler (MDI), dry powder inhaler (DPI) or nebulizer. Preferably, said compositions can be applied with state of the art nebulizers using technologies like jet nebulizer, vibrating mesh nebulizer, ultrasonic wave nebulizer, human powered nebulizer and soft mist inhaler. The topical administration to the lung with the above mentioned techniques is conducted once or multiple times per day, preferably twice daily. A single administration may take from few seconds (for a DPI or MDI) up to 30 min (for an aerosol administration with a nebulizer).

An advantageous mass concentration of the tyrosine kinase inhibitor in the above-mentioned pharmaceutical formulation is in the range from 4 mg/mL to 40 mg/mL. Advantageous mass concentrations of each of the β2-adrenoceptor agonists in the above-mentioned pharmaceutical formulation are in the range from 0.4 µg/mL to 8 µg/mL.

As another advantage of the pharmaceutical compositions of the present invention, the treatment of ILDs may be facilitated by allowing for the use of a fixed-dose combination, i.e. the application of a single medicament, and/or for the use of one single device, e.g. nebulizer or inhaler, which may improve, for instance, the ease of use, patient compliance and thus treatment success.

In a third aspect of the present invention, a pharmaceutical kit for simultaneous, sequential and/or separate use of effective doses of the above-mentioned active pharmaceutical ingredients is provided. Advantages of pharmaceutical kits are known to the one skilled in the art. Said pharmaceutical kit may encompass one or more of the active substances themselves and/or one or more of the above-mentioned pharmaceutical compositions of the present invention. In addition, said pharmaceutical kit may comprise one or more of the above-mentioned medical devices for inhalative administration. For instance, above-mentioned components of said pharmaceutical kit are provided in separate compartments of the kit.

In a forth aspect, the present disclosure describes a method for the treatment of one or more ILDs in a patient in need thereof with one or more of the above-mentioned active pharmaceutical ingredients. Furthermore, the present disclosure describes a method for the treatment of one or more ILDs with one or more of the above-mentioned pharmaceutical compositions.

### Preferred Embodiments

According to a first aspect of the present invention, a tyrosine kinase inhibitor, selected from nintedanib and pharmaceutically acceptable salts thereof, is provided for use in a method for the treatment of one or more ILDs in a patient in need thereof by coadministration with a β2-adrenoceptor agonist, selected from olodaterol and pharmaceutically acceptable salts thereof.

According to one embodiment, said tyrosine kinase inhibitor is nintedanib in the form of its monoethane-sulphonate salt.

According to another embodiment, said β2-adrenoceptor agonist is olodaterol in the form of its hydrochloride salt. According to another embodiment, said use in a method of treatment is directed towards one or more ILDs, selected from the group consisting of idiopathic pulmonary fibrosis, idiopathic non-specific interstitial pneumonia, chronic hypersensitivity pneumonitis, unclassifiable idiopathic interstitial pneumonia, interstitial pneumonia with autoimmune features, environmental and/or occupational fibrosis, asbestosis, silicosis, systemic sclerosis ILD and rheumatoid arthritis ILD,
preferably selected from the group consisting of idiopathic pulmonary fibrosis, systemic sclerosis ILD and rheumatoid arthritis ILD,
most preferably the ILD to be treated is idiopathic pulmonary fibrosis.

According to another embodiment, the tyrosine kinase inhibitor is administered orally. According to another embodiment, the oral daily dose of the tyrosine kinase inhibitor is in the range from 100 mg to 300 mg, preferably in the range from 200 mg to 300 mg, most preferably 300 mg.

According to another embodiment, the tyrosine kinase inhibitor is administered via inhalation.

According to another embodiment, the inhalative daily dose of the tyrosine kinase inhibitor is in the range the range from 10 mg to 100 mg, preferably in the range from 12.5 mg to 70 mg, most preferably in the range from 15 mg to 35 mg.

According to another embodiment, the β2-adrenoceptor agonists is coadministered via inhalation.

According to another embodiment, the inhalative daily dose of the β2-adrenoceptor agonist to be coadministered is in the range from 0.01 µg to 50 µg, preferably from 0.05 µg to 25 µg, more preferably from 1 µg to 20 µg, most preferably from 5 µg to 10 µg.

According to a second aspect, the present disclosure describes a pharmaceutical composition for inhalative administration, preferably selected from aerosol compositions, suspensions and solutions, comprising said tyrosine kinase inhibitor.

Also, according to the second aspect of the present invention, a pharmaceutical composition for inhalative administration, preferably selected from aerosol compositions, dry powders, suspensions and solutions, comprising said tyrosine kinase inhibitor and said β2-adrenoceptor agonist to be coadministered is provided.

According to one embodiment, said pharmaceutical composition for inhalative administration comprises one or more excipients that improve distribution and/or tissue penetration in the lung, preferably surface active excipients, i.e. surface tension lowering excipients, most preferably tensides and/or surfactants.

According to another embodiment, said pharmaceutical composition for inhalative administration is an aqueous, ethanolic or aqueous-ethanolic solution, most preferably an aqueous-ethanolic solution, for instance with a volume to volume ratio water:ethanol of about 4:1.

According to another embodiment, the mass concentration of the above-mentioned tyrosine kinase inhibitor in said pharmaceutical composition for inhalative administration is in the range from 4 mg/mL to 40 mg/mL, preferably in the range from 5 mg/mL to 28 mg/mL, most preferably in the range from 6 mg/mL to 14 mg/mL.

According to another embodiment, the mass concentration of said β2-adrenoceptor agonist to be coadministered in said pharmaceutical composition for inhalative administration is in the range from from 0.4 µg/mL to 8 µg/mL, preferably from 2 µg/mL to 4 µg/mL.

According to another embodiment, said pharmaceutical composition for inhalative administration is applied with a nebulizer, preferably a jet nebulizer, a vibrating mesh nebulizer, an ultrasonic wave nebulizer, a human powered nebulizer or a soft mist inhaler.

According to a third aspect of the present invention, a pharmaceutical kit is provided for simultaneous, sequential and/or separate use of the above-mentioned tyrosine kinase inhibitor and the above-mentioned β2-adrenoceptor agonist to be coadministered.

According to one embodiment, said pharmaceutical kit comprises separate compartments for each of one or more pharmaceutical compositions comprising said tyrosine kinase inhibitor and/or said β2-adrenoceptor agonist and a medical device for inhalative administration of said pharmaceutical compositions.

According to another embodiment, said pharmaceutical kit comprises
i) a first compartment containing a pharmaceutical composition for oral administration, preferably selected from solid dosage forms, comprising a therapeutically effective amount of said tyrosine kinase inhibitor,
ii) a second compartment containing a pharmaceutical composition for inhalative administration, preferably selected from aerosol compositions, dry powders, suspensions and solutions, comprising a therapeutically effective amount of said β2-adrenoceptor agonist to be coadministered,
iii) a medical device for inhalative administration of a therapeutically effective amount of the content of the second compartment,

According to another embodiment, said pharmaceutical kit comprises
i) a first compartment containing a pharmaceutical composition for inhalative administration, preferably selected from aerosol compositions, suspensions and solutions, comprising a therapeutically effective amount of said tyrosine kinase inhibitors and at least one pharmaceutically acceptable excipient,
ii) a second compartment containing a pharmaceutical composition for inhalative administration, preferably selected from aerosol compositions, dry powders, suspensions and solutions, comprising a therapeutically effective amount of said β2-adrenoceptor agonist to be coadministered,
iii) a medical device for inhalative administration of therapeutically effective amounts of the contents of the first and second compartment.

According to another embodiment, said pharmaceutical kit comprises
i) a first compartment containing a pharmaceutical composition for inhalative administration, preferably selected from aerosol compositions, dry powders, suspensions and solutions, comprising therapeutically effective amounts of said tyrosine kinase inhibitor and of said β2-adrenoceptor agonist to be coadministered,
ii) a medical device for inhalative administration of therapeutically effective amounts of the contents of the first compartment.

According to a forth aspect of the present disclosure, a method for the treatment of one or more ILDs with the above-mentioned tyrosine kinase inhibitor is described.

In particular, a method for the treatment of one or more ILDs with one or more of the above-mentioned pharmaceutical compositions is described.

### Examples and Experimental Data

The following examples are for the purpose of illustration of the invention only and are not intended in any way to limit the scope of the present invention.

### A) Clinical trial to assess the effect of combined inhalative nintedanib and olodaterol treatment on FVC decline in patients with IPF

A 12-week, double blind, randomised, placebo controlled, parallel group trial followed by a single active arm phase of 40 weeks evaluating the effect of inhaled olodaterol in combination with nintedanib on change in FVC.

Main inclusion criteria: Male or female patients aged ≥ 40 years at visit 1 (screening); IPF diagnosis based upon ATS/ERS/JRS/ALAT 2011 guideline within 3 years of visit 0; HRCT performed within 18 months of visit 0; confirmation of diagnosis by central review of chest high-resolution computed tomography (HRCT) and potentially surgical lung biopsy (later if available) prior to randomization; FVC ≥ 80% predicted of normal at visit 1 (screening).

Posology: Twice daily application as an aerosol of an aqueous-ethanolic solution 4/1 (v/v) of nintedanib at 10 mg/mL and olodaterol at 0.1 mg/mL as for 30 min with a Pari Nebulizer

### Primary Endpoint: Rate of change (slope) in FVC from baseline to week 12

Key Secondary Endpoint: Proportion of patients with disease progression as defined by absolute FVC (% predicted) decline ≥10% or death until week 52

Safety criteria: Adverse events (especially SAE and other significant AE), physical examination, weight measurements, 12 lead electrocardiogram, vital signs and laboratory evaluations.

Statistical methods: Random coefficient regression models for continuous endpoints, Log rank tests, Kaplan-Meier plots and Cox regressions for time to event endpoints, logistic regression models or other appropriate methods for binary endpoints.

### B) Method to treat connective tissue diseases-ILD like SSc-ILD or RA-ILD with a fixed dose combination of inhaled nintedanib and olodaterol

Patients: Male or female patients aged ≥ 18 years. Extent of fibrosis in the lung > 10%; FVC > 40% predicted, DLCO 30% to 89% predicted (corrected for Hb).

Diagnosis: Patients diagnosed with SSc-ILD or RA-ILD according to the most recent ACR / EULAR criteria and HRCT (within previous 12 months).

Posology: Twice daily application of a fixed dose combination of olodaterol (5 µg) and nintedanib (30 mg) as an aerosol of an aqueous phosphate buffered (0.05 M, pH 6) suspension containing 1% of a lipid mixture of dipalmitoylphosphatidylcholine (DPPC, 0.0278 M); 1-palmitoyl-2-oleoyl-sn-glycero-3-(phospho-rac-(1-glycerol)) sodium salt (POPG, 0.0072 M) and palmitic acid (PA, 0.00876 M) with a constant lipid molar ratio of 3.17(DPPC):0.822(POPG): 1(PA).

### C) Formulations for oral administration containing nintedanib

• Soft gelatin capsules containing 150 mg of nintedanib

| | | **Formulation A** | **Formulation B** | **Formulation C** |
|---|---|---|---|---|
| **Ingredients** | **Function** | **mg per capsule** | **mg per capsule** | **mg per capsule** |
| Nintedanib monoethane sulphonate | Active Ingredient | 180.60 | 180.60 | 180.60 |
| Triglycerides, Medium-chain | Carrier | 122.85 | 161.10 | 160.20 |
| Hard fat | Thickener | 114.75 | 76.50 | 76.50 |
| Lecithin | Wetting agent / Glidant | 1.80 | 1.80 | 2.70 |
| Gelatin | Film-former | 142.82 | 142.82 | 142.82 |
| Glycerol 85% | Plasticizer | 62.45 | 62.45 | 62.45 |
| Titanium dioxide | Colorant | 0.47 | 0.47 | 0.47 |
| Iron oxide A | Colorant | 0.08 | 0.08 | 0.08 |
| Iron oxide B | Colorant | 0.22 | 0.22 | 0.22 |
| **Total Capsule Weight** | | **626.04** | **626.04** | **626.04** |

• Further pharmaceutical dosage forms for oral administration comprising nintedanib are disclosed in WO 2009/147212 and in WO 2009/147220.

### D) Formulations for inhalation containing nintedanib

- A dry powder formulation for inhalation (Vartiainen et al., poster presentation at the International Colloquium of Lung and Airway Fibrosis in Dublin, September 2016) consists of nintedanib, mannitol and L-leucine in the mass ratio of 53:29:18. Drug particles coated by L-leucine nanocrystals are obtained by the aerosol flow reactor method in the form of a highly flowable and dispersible powder.
- One liquid formulation for inhalation is a solution of nintedanib (5 mg/mL) in water/ethanol 4/1 (v/v) or water/ethanol 1/1 (v/v) or water/ethanol 1/4 (v/v) or ethanol.
- Other liquid formulations for inhalation may be composed as follows:
   300.0, 500.0 or 700.0 mg of nintedanib monoethanesulphonate, as well as 10 mg benzalkonium chloride are topped up to 100 mL with purified water or water for injections and acidified with hydrochloric acid to a pH of 3.0 (optionally added with 10 mg or 15 mg disodium edetate) or
   300.0, 500.0 or 700.0 mg of nintedanib monoethanesulphonate, as well as 5 mg benzalkonium chloride are topped up to 100 mL with purified water or water for injections and acidified with hydrochloric acid to a pH of 3.0 (optionally added with 5 mg disodium edetate) or
   300.0, 500.0 or 700.0 mg of nintedanib monoethanesulphonate, as well as 15 mg benzalkonium chloride are topped up to 100 mL with purified water or water for injections and acidified with hydrochloric acid to a pH of 3.0 (optionally added with 10 mg disodium edetate).
- Another liquid formulation for inhalation is a suspension of nintedanib (10 mg/mL) in 2.5 mL of water (optionally with 0.5, 2 or 10 mg/mL polysorbate 80 and/or with 0.001, 0.005 or 0.01 % (w/w) povidone K25) or
   in 2.5 mL of mixtures of water and ethanol (water/ethanol 4/1 (v/v) or water/ethanol 1/1 (v/v) or water/ethanol 1/4 (v/v)) (optionally with 0.5, 2 or 10 mg/mL polysorbate 80 and/or with 0.001, 0.005 or 0.01 % (w/w) povidone K25) or
   in 2.5 mL of ethanol (optionally with 0.5, 2 or 10 mg/mL polysorbate 80 and/or with 0.001, 0.005 or 0.01 % (w/w) povidone K25).

### E) Formulations for inhalation containing olodaterol

- Liquid formulations for inhalation may be composed as follows:
   23.3, 24.8, 33.7, 38.4, 42.4, 46.1, 49.5, 53.8, 60.2 or 68.3 mg of olodaterol hydrochloride, as well as 10 mg benzalkonium chloride, 10 mg disodium edetate and 3 mg anhydrous citric acid are topped up to 100 ml with purified water or water for injections or
   23.3, 24.8, 33.7, 38.4, 42.4, 46.1, 49.5, 53.8, 60.2 or 68.3 mg of olodaterol hydrochloride, as well as 8 mg benzalkonium chloride, 8 mg disodium edetate and 4 mg anhydrous citric acid are topped up to 100 ml with purified water or water for injections or
   23.3, 24.8, 33.7, 38.4, 42.4, 46.1, 49.5, 53.8, 60.2 or 68.3 mg of olodaterol hydrochloride, as well as 5 mg benzalkonium chloride, 15 mg disodium edetate and 2 mg anhydrous citric acid are topped up to 100 ml with purified water or water for injections or
   23.3, 24.8, 33.7, 38.4, 42.4, 46.1, 49.5, 53.8, 60.2 or 68.3 mg of olodaterol hydrochloride, as well as 15 mg benzalkonium chloride, 10 mg disodium edetate and 2 mg anhydrous citric acid are topped up to 100 ml with purified water or water for injections.

### F) Formulations for inhalation containing nintedanib and olodaterol

- A dry powder formulation for inhalation consists of
   nintedanib monoethanesulphonate, olodaterol hydrochloride and lactose in the mass ratio of 1000:1:999 or
   nintedanib monoethanesulphonate, olodaterol hydrochloride and mannitol in the mass ratio of 1000:1:999 or
   nintedanib monoethanesulphonate, olodaterol hydrochloride and dextrose in the mass ratio of 1000:1:999 or
   nintedanib monoethanesulphonate, olodaterol hydrochloride and xylitol in the mass ratio of 1000:1:999 or
   nintedanib monoethanesulphonate, olodaterol hydrochloride and sorbitol in the mass ratio of 1000:1:999 or
   nintedanib monoethanesulphonate, olodaterol hydrochloride and maltitol in the mass ratio of 1000:1:999.
- One liquid formulation for inhalation is a solution of nintedanib (10 mg/mL) and olodaterol (0.1 mg/mL) in water/ethanol 4/1 (v/v).
- Another liquid formulation for inhalation is a solution of nintedanib (5 mg/mL) and olodaterol (0.1 mg/mL) in water/ethanol 4/1 (v/v) or water/ethanol 1/1 (v/v) or water/ethanol 1/4 (v/v) or ethanol.
- Another liquid formulation for inhalation is a suspension of nintedanib (30 mg) and olodaterol (5 µg) in an aqueous phosphate buffer (0.05 M, pH 6) containing 1 % of a lipid mixture of dipalmitoylphosphatidylcholine (DPPC, 0.0278 M), 1-palmitoyl-2-oleoyl-sn-glycero-3-(phospho-rac-(1-glycerol)) sodium salt (POPG, 0.0072 M) and palmitic acid (PA, 0.00876 M) with a constant lipid molar ratio DPPC : POPG : PA of 3.17 : 0.822 : 1.
- Other liquid formulations for inhalation may be composed as follows:
   300.0, 500.0 or 700.0 mg of nintedanib monoethanesulphonate, 23.3, 24.8, 33.7, 38.4, 42.4, 46.1, 49.5, 53.8, 60.2 or 68.3 mg of olodaterol hydrochloride are topped up to 100 ml with purified water or water for injections and acidified with citric acid to a pH of 3.0 or
   300.0, 500.0 or 700.0 mg of nintedanib monoethanesulphonate, 23.3, 24.8, 33.7, 38.4, 42.4, 46.1, 49.5, 53.8, 60.2 or 68.3 mg of olodaterol hydrochloride are topped up to 100 ml with purified water or water for injections and acidified with hydrochloric acid to a pH of 3.0
- Another liquid formulation for inhalation is a suspension of nintedanib monoethanesulphonate (10 mg/mL) and olodaterol hydrochloride (2 µg/mL) in 2.5 mL of water (optionally with 0.5, 2 or 10 mg/mL polysorbate 80 and/or with 0.001, 0.005 or 0.01 % (w/w) povidone K25) or
   in 2.5 mL of mixtures of water and ethanol (water/ethanol 4/1 (v/v) or water/ethanol 1/1 (v/v) or water/ethanol 1/4 (v/v) (optionally with 0.5, 2 or 10 mg/mL polysorbate 80 and/or with 0.001, 0.005 or 0.01 % (w/w) povidone K25) or
   in 2.5 mL of ethanol (optionally with 0.5, 2 or 10 mg/mL polysorbate 80 and/or with 0.001, 0.005 or 0.01 % (w/w) povidone K25).

## Claims

1. A tyrosine kinase inhibitor, selected from nintedanib and pharmaceutically acceptable salts thereof, for use in a method for the treatment of one or more interstitial lung diseases in a patient in need thereof by coadministration with a β2-adrenoceptor agonist, selected from olodaterol and pharmaceutically acceptable salts thereof.

2. The inhibitor for use according to claim 1 wherein the inhibitor is nintedanib in the form of its monoethanesulphonate salt.

3. The inhibitor for use according to one or more of claims 1 to 2 wherein the β2-adrenoceptor agonist is olodaterol in the form of its hydrochloride salt.

4. The inhibitor for use according to one or more of claims 1 to 3 wherein the one or more interstitial lung diseases are selected from the group consisting of idiopathic pulmonary fibrosis, systemic sclerosis ILD and rheumatoid arthritis ILD.

5. A pharmaceutical composition for inhalative administration, selected from aerosol compositions, dry powders, suspensions and solutions, comprising a tyrosine kinase inhibitor, selected from nintedanib and pharmaceutically acceptable salts thereof, and a β2-adrenoceptor agonist, selected from olodaterol and pharmaceutically acceptable salts thereof.

6. A pharmaceutical composition according to claim 5, comprising one or more excipients, selected from tensides and surfactants.

7. A pharmaceutical composition according to one or more of claims 5 to 6 wherein the composition is an ethanolic and/or aqueous solution.

8. A pharmaceutical kit which comprises
i) a first compartment containing a pharmaceutical composition for oral administration, selected from solid dosage forms, comprising a therapeutically effective amount of a tyrosine kinase inhibitor, selected from nintedanib and pharmaceutically acceptable salts thereof,
ii) a second compartment containing a pharmaceutical composition for inhalative administration, selected from aerosol compositions, dry powders, suspensions and solutions, comprising a therapeutically effective amount of a β2-adrenoceptor agonist, selected from olodaterol and pharmaceutically acceptable salts thereof,
iii) a medical device for inhalative administration of a therapeutically effective amount of the content of the second compartment,
for simultaneous, sequential and/or separate use of said active ingredients.

9. A pharmaceutical kit which comprises
i) a first compartment containing a pharmaceutical composition for inhalative administration, selected from aerosol compositions, suspensions and solutions, comprising a tyrosine kinase inhibitor, selected from nintedanib and pharmaceutically acceptable salts thereof, and at least one pharmaceutically acceptable excipient,
ii) a second compartment containing a pharmaceutical composition for inhalative administration, selected from aerosol compositions, dry powders, suspensions and solutions, comprising therapeutically effective amounts of a β2-adrenoceptor agonist, selected from olodaterol and pharmaceutically acceptable salts thereof,
iii) a medical device for inhalative administration of therapeutically effective amounts of the contents of the first and second compartment, for simultaneous, sequential and/or separate use of said active ingredients.

10. A pharmaceutical kit which comprises
i) a first compartment containing a pharmaceutical composition for inhalative administration, selected from aerosol compositions, dry powders, suspensions and solutions, comprising a tyrosine kinase inhibitor, selected from nintedanib and pharmaceutically acceptable salts thereof, and a β2-adrenoceptor agonist, selected from olodaterol and pharmaceutically acceptable salts thereof,
ii) a medical device for inhalative administration of therapeutically effective amounts of the contents of the first compartment,
for simultaneous, sequential and/or separate use of said active ingredients.

## Patentansprüche

1. Tyrosinkinase-Inhibitor, ausgewählt aus Nintedanib und pharmazeutisch akzeptablen bzw.
annehmbaren Salzen hiervon, zur Verwendung in einem Verfahren zur Behandlung von einer oder mehreren interstitiellen Lungenerkrankungen bei einem Patienten mit Bedarf hierfür durch Co-Verabreichung mit einem β2-Adrenozeptoragonisten, ausgewählt aus Olodaterol und pharmazeutisch akzeptablen bzw. annehmbaren Salzen hiervon.

2. Inhibitor zur Verwendung nach Anspruch 1, wobei der Inhibitor Nintedanib in Form seines Monoethansulfonatsalzes darstellt.

3. Inhibitor zur Verwendung nach einem oder mehreren der Ansprüche 1 bis 2, wobei der β2-Adrenozeptoragonist Olodaterol in Form seines Hydrochloridsalzes darstellt.

4. Inhibitor zur Verwendung nach einem oder mehreren der Ansprüche 1 bis 3, wobei die eine oder mehreren interstitiellen Lungenerkrankungen ausgewählt sind aus der Gruppe, bestehend aus idiopathischer pulmonaler Fibrose, systemischer Sklerose ILD und rheumatoider Arthritis ILD.

5. Pharmazeutische Zusammensetzung zur inhalativen Verabreichung, ausgewählt aus Aerosolzusammensetzungen, Trockenpulvern, Suspensionen und Lösungen, umfassend einen Tyrosinkinase-Inhibitor, ausgewählt aus Nintedanib und pharmazeutisch akzeptablen bzw. annehmbaren Salzen hiervon, und einen β2-Adrenozeptoragonisten, ausgewählt aus Olodaterol und pharmazeutisch akzeptablen bzw. annehmbaren Salzen hiervon.

6. Pharmazeutische Zusammensetzung nach Anspruch 5, umfassend einen oder mehrere Hilfsstoffe, ausgewählt aus Tensiden und oberflächenaktiven Mitteln.

7. Pharmazeutische Zusammensetzung nach einem oder mehreren der Ansprüche 5 bis 6, wobei die Zusammensetzung eine ethanolische und/oder wässerige Lösung darstellt.

8. Pharmazeutisches Kit, welches umfasst:
i) ein erstes Kompartement bzw. Fach, enthaltend eine pharmazeutische Zusammensetzung zur oralen Verabreichung, ausgewählt aus festen Dosierungsformen, umfassend eine therapeutisch wirksame Menge eines Tyrosinkinase-Inhibitors, ausgewählt aus Nintedanib und pharmazeutisch akzeptablen bzw. annehmbaren Salzen hiervon,
ii) ein zweites Kompartement bzw. Fach, enthaltend eine pharmazeutische Zusammensetzung zur inhalativen Verabreichung, ausgewählt aus Aerosolzusammensetzungen, Trockenpulvern, Suspensionen und Lösungen, umfassend eine therapeutisch wirksame Menge eines β2-Adrenozeptoragonisten, ausgewählt aus Olodaterol und pharmazeutisch akzeptablen bzw. annehmbaren Salzen hiervon,
iii) eine medizinische Vorrichtung zur inhalativen Verabreichung einer therapeutisch wirksamen Menge des Inhalts des zweiten Kompartements bzw. Fachs,
zur gleichzeitigen, aufeinander folgenden und/oder getrennten Verwendung der wirksamen Bestandteile.

9. Pharmazeutisches Kit, welches umfasst:
i) ein erstes Kompartement bzw. Fach, enthaltend eine pharmazeutische Zusammensetzung zur inhalativen Verabreichung, ausgewählt aus Aerosolzusammensetzungen, Suspensionen und Lösungen, umfassend einen Tyrosinkinase-Inhibitor, ausgewählt aus Nintedanib und pharmazeutisch akzeptablen bzw. annehmbaren Salzen hiervon, und mindestens einen pharmazeutisch akzeptablen bzw. annehmbaren Hilfsstoff,
ii) ein zweites Kompartement bzw. Fach, enthaltend eine pharmazeutische Zusammensetzung zur inhalativen Verabreichung, ausgewählt aus Aerosolzusammensetzungen, Trockenpulvern, Suspensionen und Lösungen, umfassend therapeutisch wirksame Mengen eines β2-Adrenozeptoragonisten, ausgewählt aus Olodaterol und pharmazeutisch akzeptablen bzw. annehmbaren Salzen hiervon,
iii) eine medizinische Vorrichtung zur inhalativen Verabreichung von therapeutisch wirksamen Mengen des Inhalts des ersten und zweiten Kompartements bzw. Fachs zur gleichzeitigen, aufeinander folgenden und/oder getrennten Verwendung der wirksamen Bestandteile.

10. Pharmazeutisches Kit, welches umfasst:
i) ein erstes Kompartement bzw. Fach, enthaltend eine pharmazeutische Zusammensetzung zur inhalativen Verabreichung, ausgewählt aus Aerosolzusammensetzungen, Trockenpulvern, Suspensionen und Lösungen, umfassend einen Tyrosinkinase-Inhibitor, ausgewählt aus Nintedanib und pharmazeutisch akzeptablen bzw. annehmbaren Salzen hiervon, und einen β2-Adrenozeptoragonisten, ausgewählt aus Olodaterol und pharmazeutisch akzeptablen bzw. annehmbaren Salzen hiervon,
ii) eine medizinische Vorrichtung zur inhalativen Verabreichung von therapeutisch
wirksamen Mengen des Inhalts des ersten Kompartements bzw. Fachs zur gleichzeitigen, aufeinander folgenden und/oder getrennten Verwendung der wirksamen Bestandteile.

## Revendications

1. Inhibiteur de la tyrosine kinase, sélectionné parmi le nintédanib et des sels pharmaceutiquement acceptables de celui-ci, pour son utilisation dans une méthode pour le traitement d'une ou de plusieurs maladies pulmonaires interstitielles chez un patient en ayant besoin par administration conjointe avec un agoniste de l'adrénocepteur β2, sélectionné parmi l'olodatérol et des sels pharmaceutiquement acceptables de celui-ci.

2. Inhibiteur pour son utilisation selon la revendication 1, dans lequel l'inhibiteur est le nintédanib sous la forme de son sel monoéthanesulfonate.

3. Inhibiteur pour son utilisation selon une ou plusieurs des revendications 1 à 2, dans lequel l'agoniste de l'adrénocepteur β2 est l'olodatérol sous la forme de son sel de chlorhydrate.

4. Inhibiteur pour son utilisation selon une ou plusieurs des revendications 1 à 3, dans lequel la ou les maladies pulmonaires interstitielles sont sélectionnées parmi le groupe constitué de la fibrose pulmonaire idiopathique, la sclérose MPI systémique et la polyarthrite rhumatoïde MPI.

5. Composition pharmaceutique pour une administration par inhalation, choisie parmi des compositions d'aérosol, des poudres sèches, des suspensions et des solutions, comprenant un inhibiteur de la tyrosine kinase, sélectionné parmi le nintédanib et des sels pharmaceutiquement acceptables de celui-ci, et un agoniste de l'adrénocepteur β2, sélectionné parmi l'olodatérol et des sels pharmaceutiquement acceptables de celui-ci.

6. Composition pharmaceutique selon la revendication 5, comprenant un ou plusieurs excipients, sélectionnés parmi des tensides et des agents de surface.

7. Composition pharmaceutique selon une ou plusieurs des revendications 5 à 6, dans laquelle la composition est une solution éthanolique et/ou aqueuse.

8. Kit pharmaceutique qui comprend :
i) un premier compartiment contenant une composition pharmaceutique pour une administration orale, sélectionnée parmi des formes posologiques solides, comprenant une quantité thérapeutiquement efficace d'un inhibiteur de la tyrosine kinase, sélectionné parmi le nintédanib et des sels pharmaceutiquement acceptables de celui-ci,
ii) un deuxième compartiment contenant une composition pharmaceutique pour une administration par inhalation, sélectionnée parmi des compositions d'aérosol, des poudres sèches, des suspensions et des solutions, comprenant une quantité thérapeutiquement efficace d'un agoniste de l'adrénocepteur β2, sélectionné parmi l'olodatérol et des sels pharmaceutiquement acceptables de celui-ci,
iii) un dispositif médical pour une administration par inhalation d'une quantité thérapeutiquement efficace du contenu du deuxième compartiment,
pour une utilisation simultanée, séquentielle et/ou séparée desdits ingrédients actifs.

9. Kit pharmaceutique qui comprend :
i) un premier compartiment contenant une composition pharmaceutique pour une administration par inhalation, sélectionnée parmi des compositions d'aérosol, des suspensions et des solutions, comprenant un inhibiteur de la tyrosine kinase, sélectionné parmi le nintédanib et des sels pharmaceutiquement acceptables de celui-ci, et au moins un excipient pharmaceutiquement acceptable,
ii)un deuxième compartiment contenant une composition pharmaceutique pour une administration par inhalation, sélectionnée parmi des compositions d'aérosol, des poudres sèches, des suspensions et des solutions, comprenant des quantités thérapeutiquement efficaces d'un agoniste de l'adrénocepteur β2, sélectionné parmi l'olodatérol et des sels pharmaceutiquement acceptables de celui-ci,
iii) un dispositif médical pour une administration par inhalation de quantités thérapeutiquement efficaces du contenu du premier et du deuxième compartiment, pour une utilisation simultanée, séquentielle et/ou séparée desdits ingrédients actifs.

10. Kit pharmaceutique qui comprend :
i) un premier compartiment contenant une composition pharmaceutique pour une administration par inhalation, sélectionnée parmi des compositions d'aérosol, des poudres sèches, des suspensions et des solutions, comprenant un inhibiteur de la tyrosine kinase, sélectionné parmi le nintédanib et des sels pharmaceutiquement acceptables de celui-ci, et un agoniste de l'adrénocepteur β2, sélectionné parmi l'olodatérol et des sels pharmaceutiquement acceptables de celui-ci,
ii) un dispositif médical pour une administration par inhalation de quantités thérapeutiquement efficaces du contenu du premier compartiment,
pour une utilisation simultanée, séquentielle et/ou séparée desdits ingrédients actifs.
